# EUROPEAN PATENT APPLICATION

(11) **EP 2 158 918 A1**
(43) Date of publication of application: **03.03.2010**
(21) Application number: 09168624.6
(22) Date of filing: 25.08.2009
(51) Int. Cl.: A61K 38/01, A61K 35/20, A61P 3/08

(54) **Method for reducing postprandial glucose levels with a composition of whey protein**

(30) Priority: 29.08.2008 US 93037 P
(71) Applicant: Kraft Foods Global Brands LLC, Northfield, IL 60093 (US); THE GOVERNING COUNCIL OF THE UNIVERSITY OF TORONTO, Toronto, Ontario M5S 1A1 (CA)
(72) Inventor: Brown, Peter Harris, Glenview, IL 60025 (US); Matusheski, Nathan, Gurnee, IL 60031 (US); Rubin, Martyn, Chicago, IL 60657 (US); Anderson, Gerald, Toronto Ontario M5R 1C6 (CA)
(74) Representative: Murray, Adrian D'Coligny

(57) **Abstract**

A method is provided that is effective for reducing postprandial blood glucose levels and/or food intake levels. The method includes administering native whey protein to a subject in an amount and at a time prior to a meal that is effective for reducing postprandial blood glucose levels and/or reducing food intake.

## Description

This application claims the benefit of U.S. Provisional Application No. 61/093,037, filed August 29, 2008, which is incorporated in its entirety herein by reference.

The present invention relates to a method for reducing postprandial blood glucose levels and/or a method for reducing food intake. More particularly, the method includes administering native whey protein to a subject in an amount and at a time prior to a meal that is effective for reducing postprandial blood glucose levels and/or reducing food intake.

### BACKGROUND

There is significant global growth of diabetes and pre-diabetes, the defining characteristic of which is chronic hyperglycemia. Following a meal, blood glucose levels in these individuals' and individuals at potential risk for diabetes increases beyond "normal" levels and remains high for a longer period of time. For diabetics, the previous solution has been the combination of drug therapy and a diet of low "glycemic" foods, that is, foods high in dietary fiber and low in fat and digestible carbohydrates (i.e., simple sugars and stanch). For pre-diabetics and those who may be at risk for becoming diabetic by displaying postprandial hyperglycemia, the previous solution has been a diet of low glycemic foods.

### SUMMARY

A method is provided for controlling postprandial blood glucose levels by administration of specific amounts of whey protein within a specific window of time prior to a meal. Ingestion of whey protein at certain times before a meal (i.e., a whey protein pre-load) significantly reduces postprandial (after a meal) blood glucose levels and the kinetics of glucose appearance in the blood. The method is effective for maintaining a more normal blood glucose homeostasis.

More specifically, a method is provided for reducing postprandial blood glucose levels. The method includes administering from about 2 to about 90 grams, in another aspect about 10 to about 90 grams, in another aspect about 2 to about 40 grams, and in another aspect about 10 to about 40 grams of native whey protein to a subject. The native whey protein is administered to the subject at least about 5 to about 90 minutes, in another aspect about 20 to about 40 minutes, and preferably about 30 minutes prior to a meal. The method is effective for reducing postprandial blood glucose levels by at least about 5% as compared to the same subject not administered native whey protein prior to a meal.

In an important aspect of the invention, native whey has a level of hydrolysis of about 2% or less. Native whey protein that may be utilized includes sweet whey, acid whey, individual whey proteins and mixture thereof.

A method is also provided for reducing food intake in a subject. The method includes administering from about 2 to about 90 grams, in another aspect about 10 to about 90 grams, in another aspect about 2 to about 40 grams, and in another aspect about 10 to about 40 grams of native whey protein to a subject at least about 5 to about 90 minutes, in another aspect about 20 to about 40 minutes, and preferably about 30 minutes prior ta a meal. The method is effective for reducing next meal caloric intake by at least about 10% and as compared to a subject not administered native whey protein prior to a meal.

In another aspect, a method is provided for reducing postprandial blood glucose levels and/or food intake that includes administering an edible composition to a subject, where the edible composition includes whey protein. The edible composition may include a beverage, a nutrition supplement, a food composition, a meal replacement product, and mixtures thereof.

### BRIEF DESCRIPTION OF THE DRAWINGS

Figure 1 illustrates the effect of feeding a whey protein pre-load dose on ad libitum food intake response.

Figure 2 shows a comparison of blood glucose concentrations pre- and post-ad libitum meal of a whey protein pre-load dose response study.

Figure 3 illustrates changes in blood glucose concentrations pre- and post-ad libitum meal of a whey protein pre-load dose response study.

Figure 4 shows blood glucose AUC (area under curve) pre- and post-ad libitum meal of a whey protein pre-load dose response study.

Figure 5 illustrates average appetite pre- and post-ab libitum meal of a whey protein pre-load dose response study.

Figure 6 shows average appetite AUC pre- and post ad libitum meal of a whey protein pre-load dose response study.

Figure 7 illustrates average appetite per gram whey protein pre- and post-ad libitum meal of a whey protein pre-load dose response study.

Figure 8 shows blood glucose concentrations pre- and post-meal in fixed meal (12 kcal/kg body weight) whey protein pre-load dose response study.

Figure 9 shows changes in blood glucose concentrations pre- and post-meal in fixed meal (12 kcal/kg body weight) whey protein pre-load dose response study.

Figure 10 shows blood glucose AUC pre- and post-meal in fixed meal (12 kcal/kg body weight) whey protein pre-load dose response study.

Figure 11 illustrates post-meal blood glucose AUC (30-170 minutes) in fixed meal (12 kcal/kg body weight) whey protein pre-load dose response study.

Figure 12 shows insulin concentrations pre- and post-meal in fixed meal (12 kcal/kg body weight) whey protein pre-load dose response study.

Figure 13 illustrates changes in insulin concentrations pre- and post-meal in fixed meal (12 kcal/kg body weight) whey protein pre-load dose response study.

Figure 14 shows insulin AUC pre- and post-meal in fixed meal (12 kcal/kg body weight) whey protein pre-load dose response study.

Figure 15 illustrates changes in insulin concentration directly prior to the meal (30 min), and post-meal (110 min) in fixed meal (12 kcal/kg body weight) whey protein pre-load dose response study.

Figure 16 illustrates average appetite scores pre- and post-meal in fixed meal (12 kcal/kg body weight) whey protein pre-load dose response study.

Figure 17 shows differences in average appetite scores pre- and post-meal in fixed meal (12 kcal/kg body weight) whey protein pre-load dose response study.

Figure 18 illustrates average appetite AUC pre- and post-meal in fixed meal (12 kcal/kg body weight) whey protein pre-load dose response study.

Figure 19 illustrates feeling of fatigue AUC post-meal (30-170 minutes) in fixed meal (12 kcal/kg body weight) whey protein pre-load dose response study.

Figure 20 illustrates correlations between pre-meal and post-meal insulin AUC as a function of either (20a) pre-meal insulin AUC or (20b) post-meal blood glucose AUC in fixed meal (12 kcal/kg body weight) whey protein pre-load dose response study.

Figure 21 shows correlations between post-meal blood glucose AUC and post-meal insulin AUC in fixed meal (12 kcal/kg body weight) whey protein pre-load dose response study.

Figure 22 illustrates post-meal blood glucose iAUC (0-120 minutes).

Figure 23 illustrates post-meal blood insulin iAUC (0-120 minutes).

### DETAILED DESCRIPTION

A method is provided that is effective for reducing postprandial blood glucose levels and/or food intake levels. Accordingly, the method may promote satiety, which may lead to weight loss and lowering of blood glucose levels. In accordance with the method, a native whey protein is administered to a subject directly or as part of an edible composition. Administration of the native whey protein is effective for reducing postprandial blood glucose levels by at least about 5%, preferably at least about 20% and most preferably at least about 50% as compared to the same subject not administered native whey protein prior to a meal. In another aspect, the method is effective for reducing caloric intake by at least about 9%, preferably at least about 14%, and most preferably at least about 27% as compared to the same subjected not administered native whey protein prior to a meal.

### Native Whey Protein

Native whey proteins that may be utilized include sweet whey, acid whey, individual whey proteins (i.e., a-lacalbumin, b-lactoglobulin, albumin, immunoglobulins, glycomaropeptide derived from casein, lactoferrin), and mixture thereof. Suitable commercial sources of native whey protein may be utilized. An example of a suitable commercial source of whey protein includes Alacen 392 whey protein concentrate (Fonterra) which is manufactured from fresh sweet cheese whey using an ultrafiltration process.

The native whey protein utilized has a very low level of hydrolysis. As used herein, "native whey protein" refers to whey protein that has a degree of hydrolysis of less than about 2%, in another aspect less than about 1%, and in another aspect, less than about 0.4% (as determined by OPA methodology, Lee et al. Int. J. Biochem. 1978; 9(7):457-467, which is incorporated herein by reference).

### Edible Composition

The edible composition may be in the form of a nutritional supplement (such as a tablet, powder, capsule or liquid product), a food composition (product), a beverage, or a meal replacement product.

A nutritional supplement as used herein refers to a composition or supplement which provides at least one beneficial agent such as vitamins, minerals, trace elements, phytochemicals, which is intended to supplement the amount of such agents obtained through normal dietary intake. These compositions or supplements do not generally contain a significant amount of calories, protein, carbohydrate or fat. They are not intended to be taken as a food but rather as a supplement to the daily diet.

A food composition may be any food which can be formulated to include native whey protein. Food compositions may include dairy based products (such as milk based products including hard and fresh cheese and yogurt), soy based products, breads and cereal based products (including pasta and cereal bars), cakes, cookies, biscuits, spreads, oil-in-water emulsions (such as dressings, ketchup and mayonnaise), ice creams, desserts, soups, powdered soup concentrates, sauces, powdered sauce concentrates, health bars, confectionery, snack foods, ready-to-eat meal products, pre-packed meal products, and dried meal products etc.

A beverage may be any beverage which can be formulated to include native whey protein. Beverages may include water, fruit juice, a low calorie fruit flavored beverage (for example, Kool-Aid, Crystal Light, powdered and ready to drink soft drinks, sport drinks), coffee, tea, smoothie, a dairy beverage, carbonated beverages, and mixtures thereof.

A meal replacement product as used herein refers to a product which is intended to replace one or more conventional meals a day; they are of a controlled calorie content and are generally eaten as a single product. However several such products may be eaten together. Examples of meal replacement products and products to be used as part of a meal replacement plan include; (ready-to-drink) liquid products such as milk or soy-based drinks, soluble powders used to prepare those drinks and drinks prepared therefrom, bars, soups, cereal or noodle or pasta-based products, desserts such as rice puddings, custards and the like and porridge and the like. Meal replacement products are generally used by consumers following a calorie controlled diet or wishing to control their body weight.

The edible composition may be for example; a solid product, a powdered product, a tablet, a capsule, a liquid, a flowable, spoonable, pourable or spreadable product or a bar etc. The edible composition may be a powder which is mixed with a liquid, such as water or milk, to produce a liquid or slurry product such as a meal replacement product, or a product to be used as part of a meal replacement plan.

### EXAMPLE 1: Dose Response

All treatment were served in liquid form (300 ml total) that included water, sucralose, unsweetened drink mix for color and flavor, and the amounts of sweet whey protein indicated below. An additional 100 ml of water was served with each treatment.

### Treatment

| No. | Treatment |
|---|---|
| 1 | 10 grams whey protein |
| 2 | 20 grams whey protein |
| 3 | 30 grams whey protein |
| 4 | 40 grams whey protein |
| 5 | Water (control) |

Treatment subjects included 16 males having an average age of 22.3 (± 0.6), an average body weight of 69.5 kg (± 1.6) and an average body mass index (BMI) of 22.6 (± 0.4).

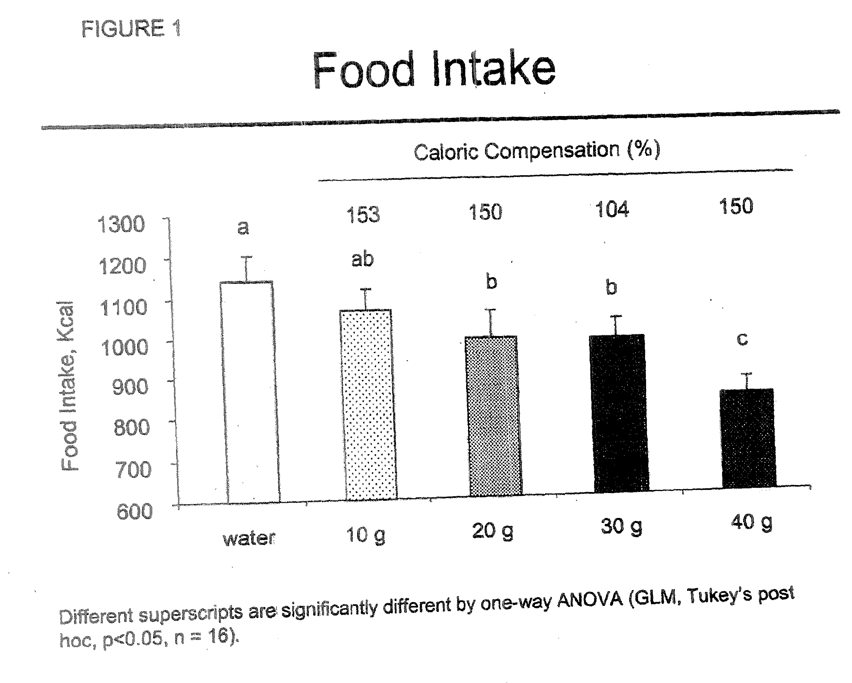

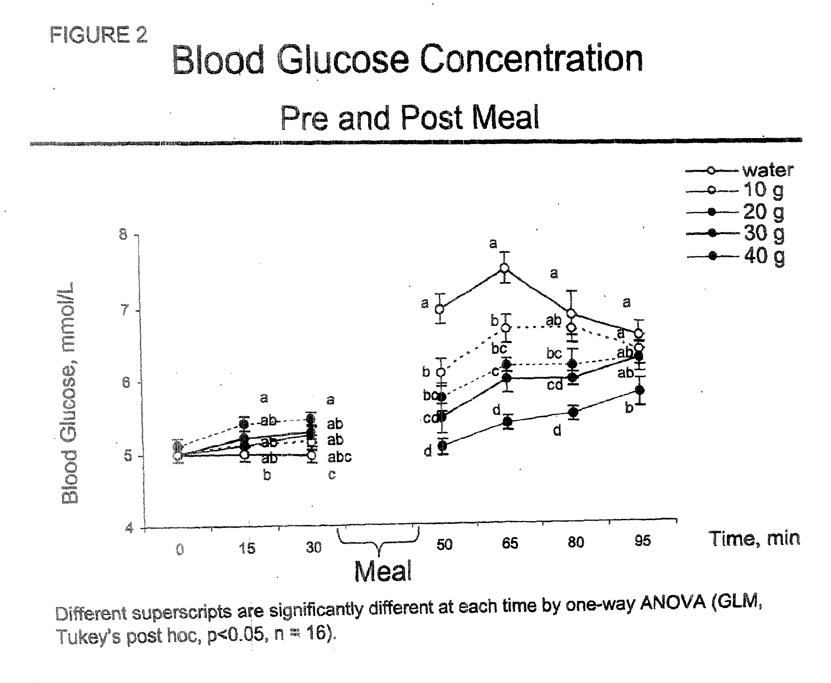

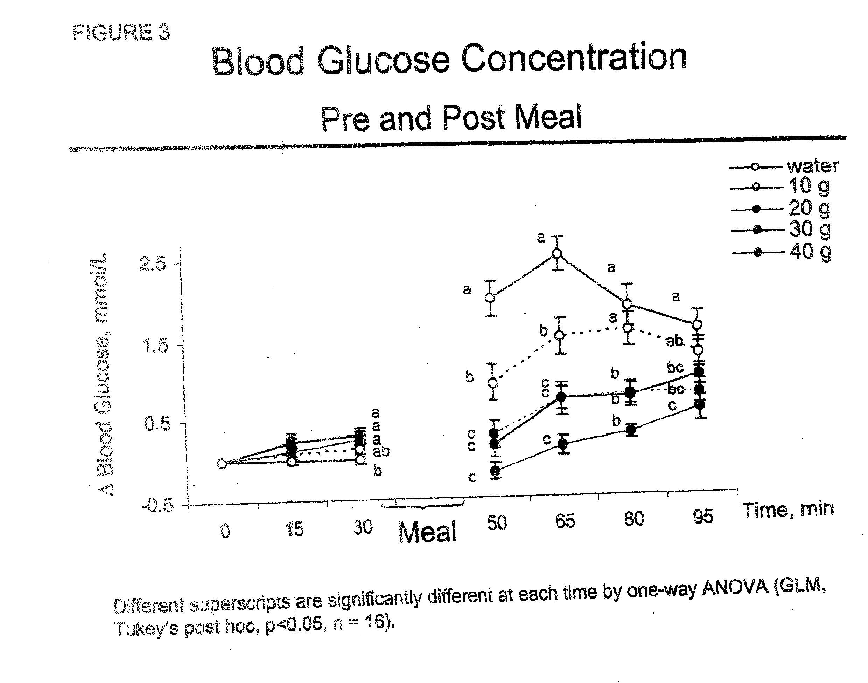

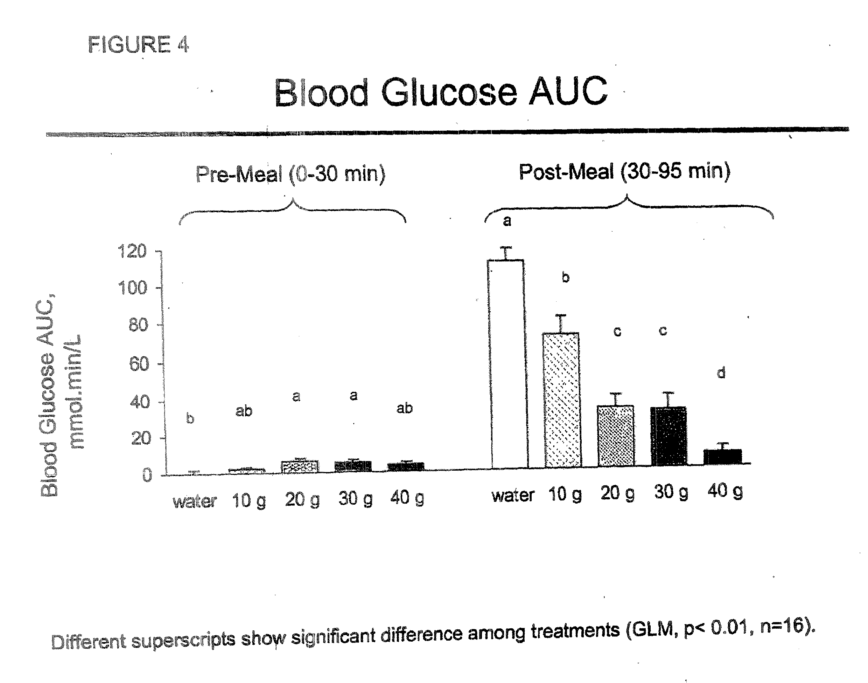

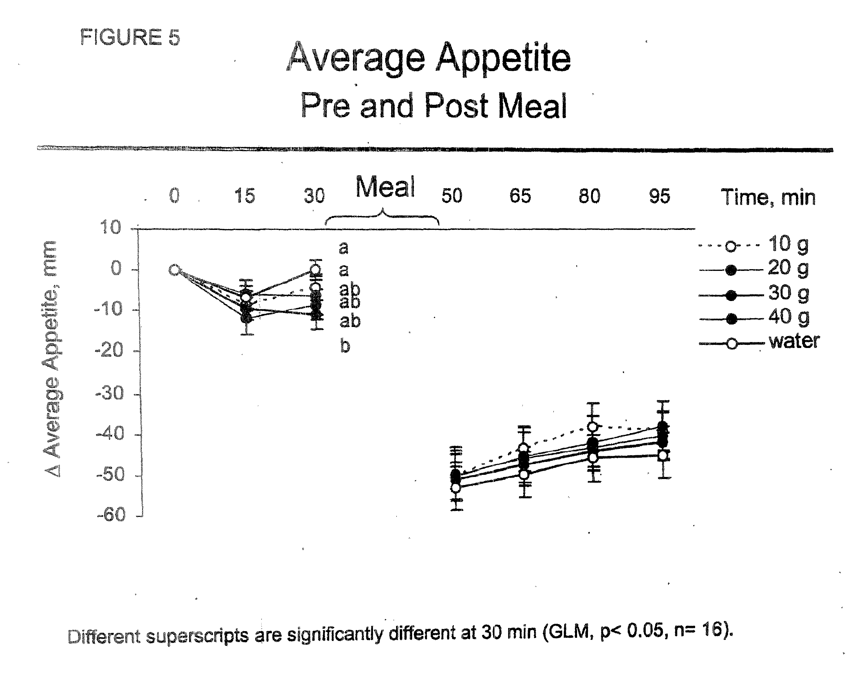

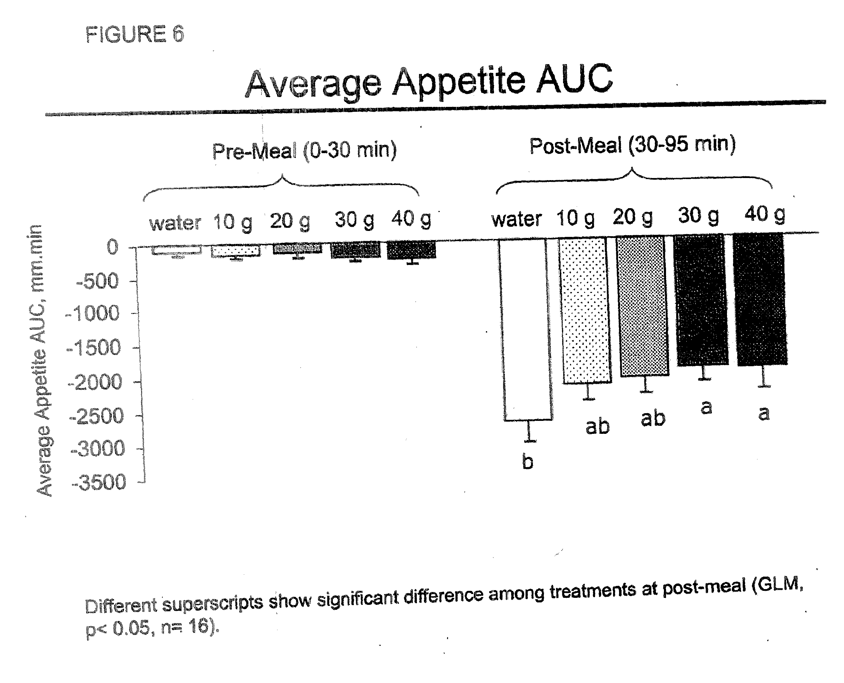

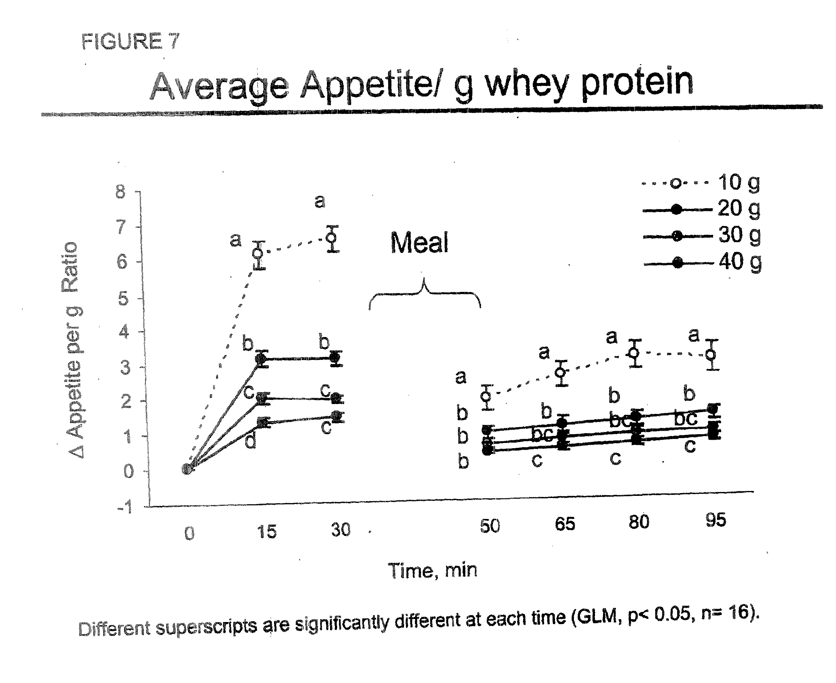

Treatment subjects randomly received the treatments at 4 hours after a standard breakfast (standard breakfast = 2% milk, orange juice and cereal for a total of 340 Kcal). Average appetite by visual analog scale (Visual Analogue Questionaire, Hill AJ, Magson LD, Blundell JE. Hunger and palatability: tracking ratings of subjective experience before, during and after the consumption of preferred and less preferred food. Appetite. 1984 Dec;5(4):361-71; and Flint A, Raben A, Blundell JE, Astrup A. Reproducibility, power and validity of visual analogue scales in assessment of appetite sensations in single test meal studies. Int J Obes Relat Metab Disord 2000;24(1):38-48, both of which are incorporated herein by reference) and blood glucose by finger prick blood collection were measured immediately before and at 15 and 30 minutes after receiving the treatments. Subjects then had 20 minutes to eat ad libitum pizza test meals. At 50 minutes (right after eating the test meal), and 65, 80 and 95 minutes after receiving the treatments, blood glucose and subjective appetite were measured. The effect of all treatments on food intake and blood glucose are illustrated in Figures 1-7.

### EXAMPLE 2: Fixed Meal Study

All treatment were served in liquid form (300 ml total) that included water, sucralose, unsweetened drink mix for color and flavor, and the amounts of sweet whey protein indicated below. An additional 100 ml of water was served with each treatment.

### Treatment

| No. | Treatment |
|---|---|
| 1 | 5 grams whey protein |
| 2 | 10 grams whey protein |
| 3 | 20 grams whey protein |
| 4 | 40 grams whey protein |
| 5 | 10 grams hydrolyzed whey protein |
| 6 | water (control) |

Treatment subjects included males between 20 and 30 years old with a BMI between 20 and 24.9.

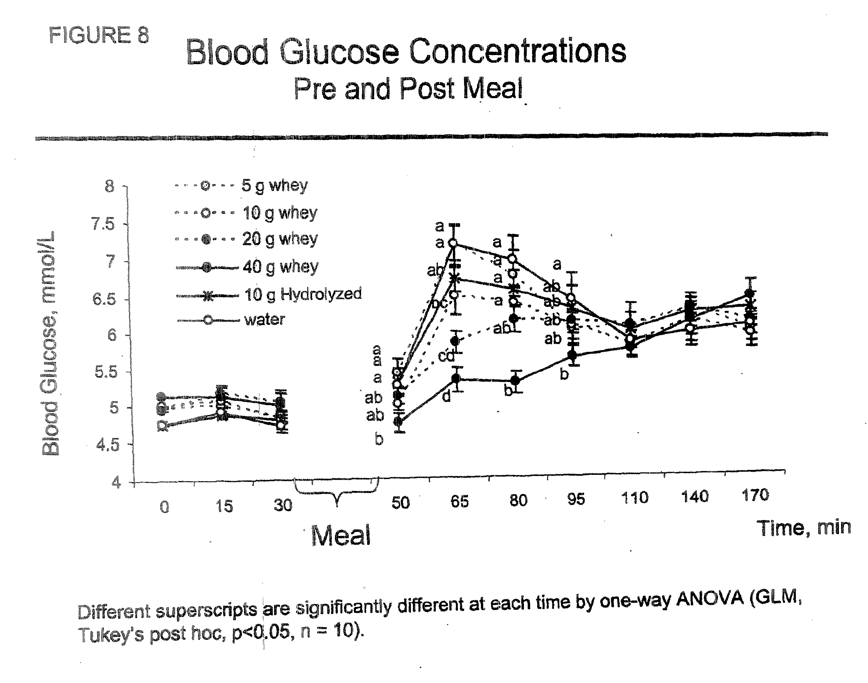

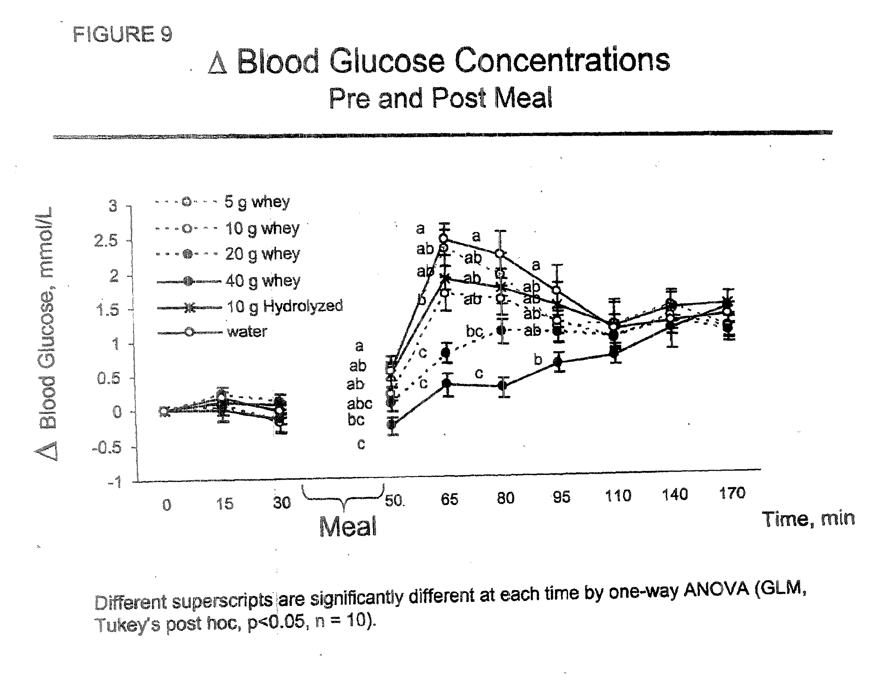

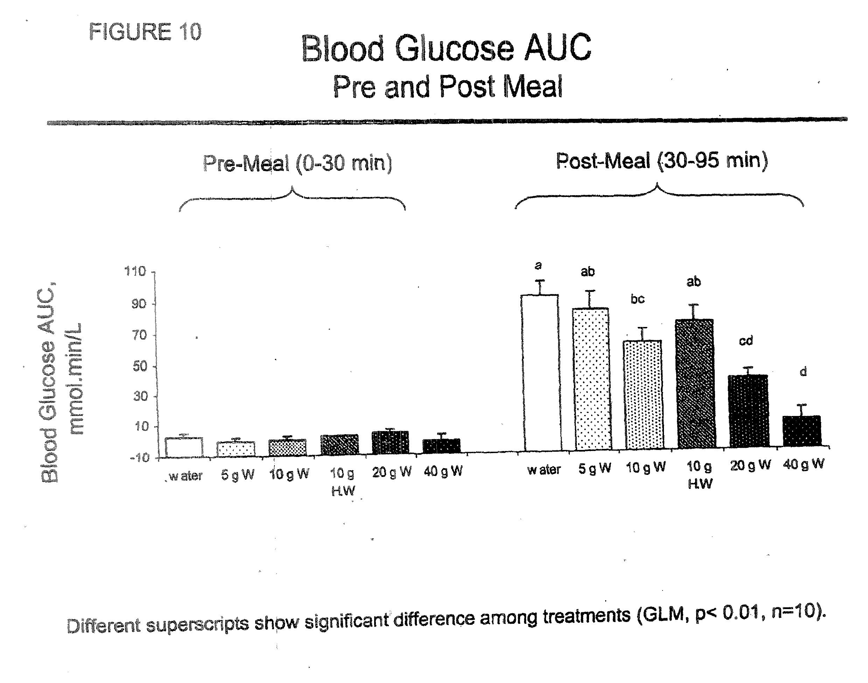

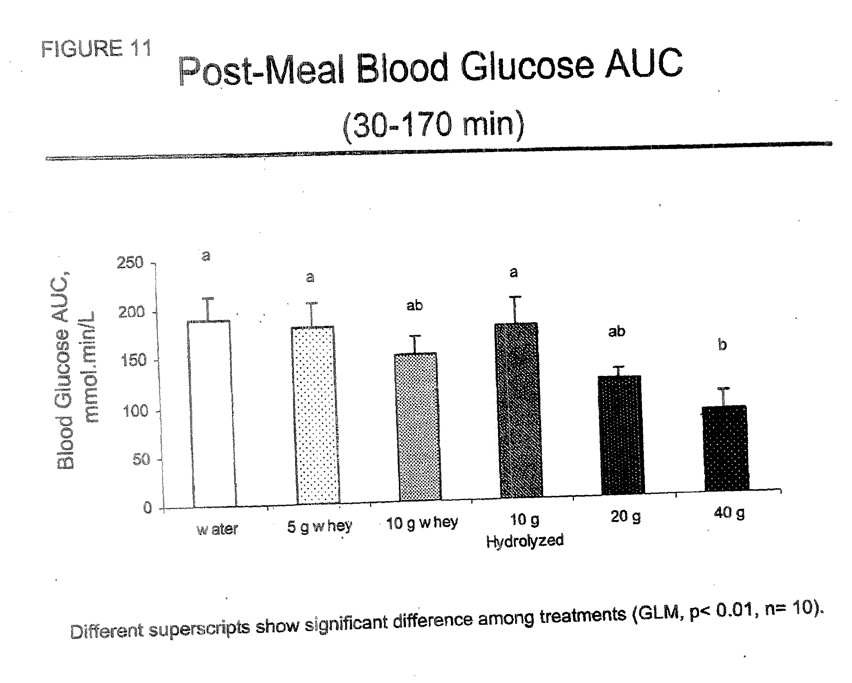

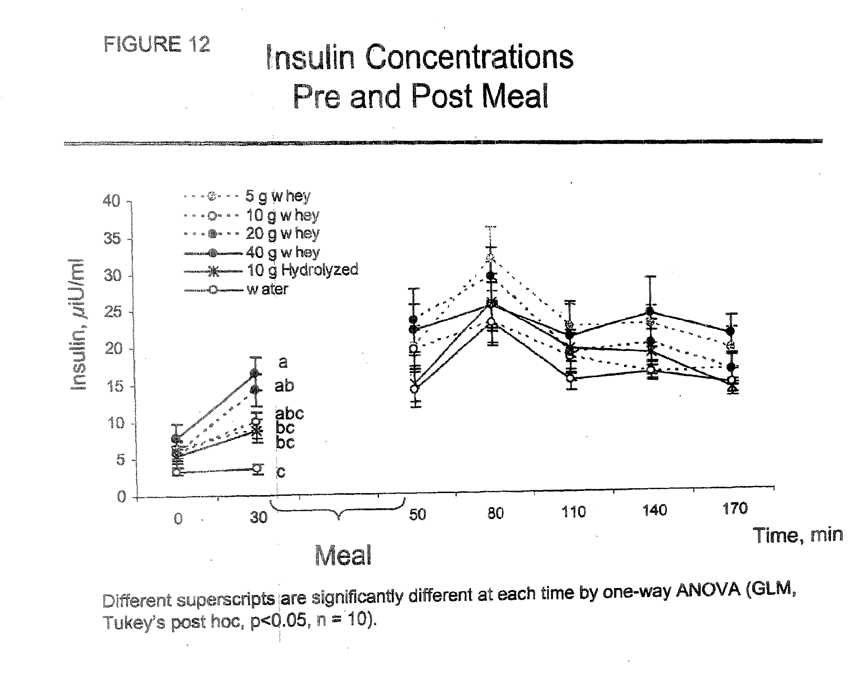

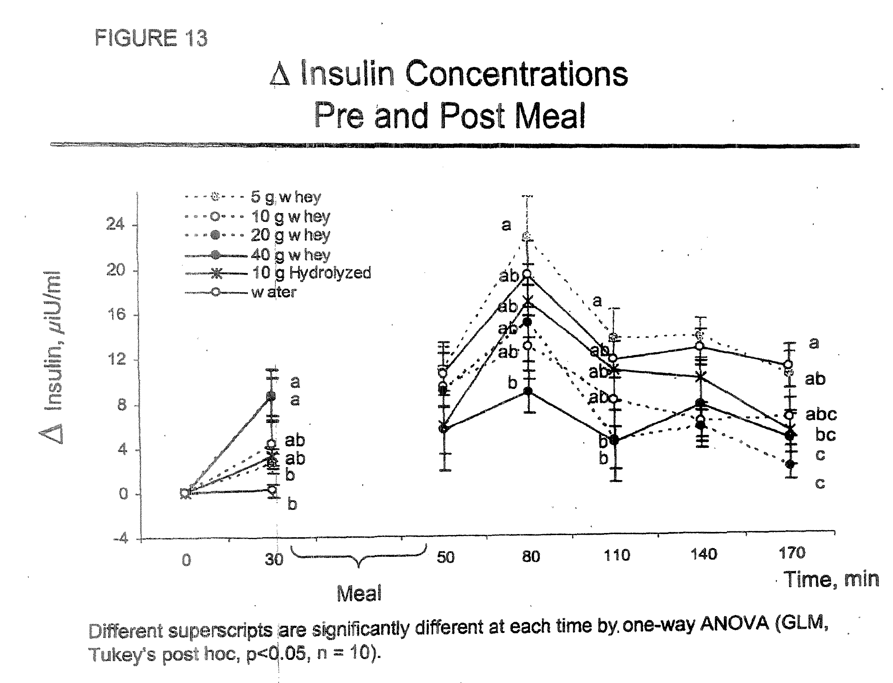

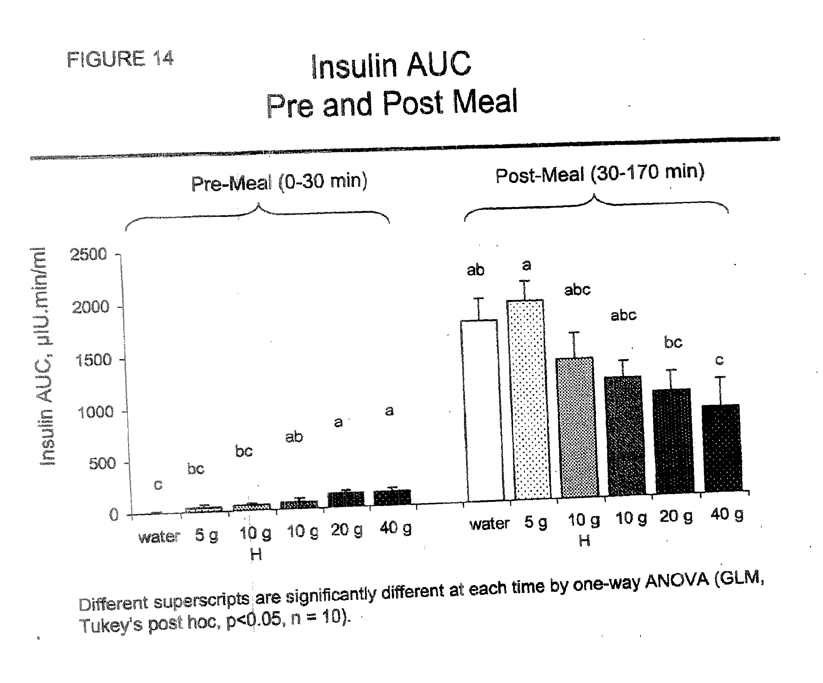

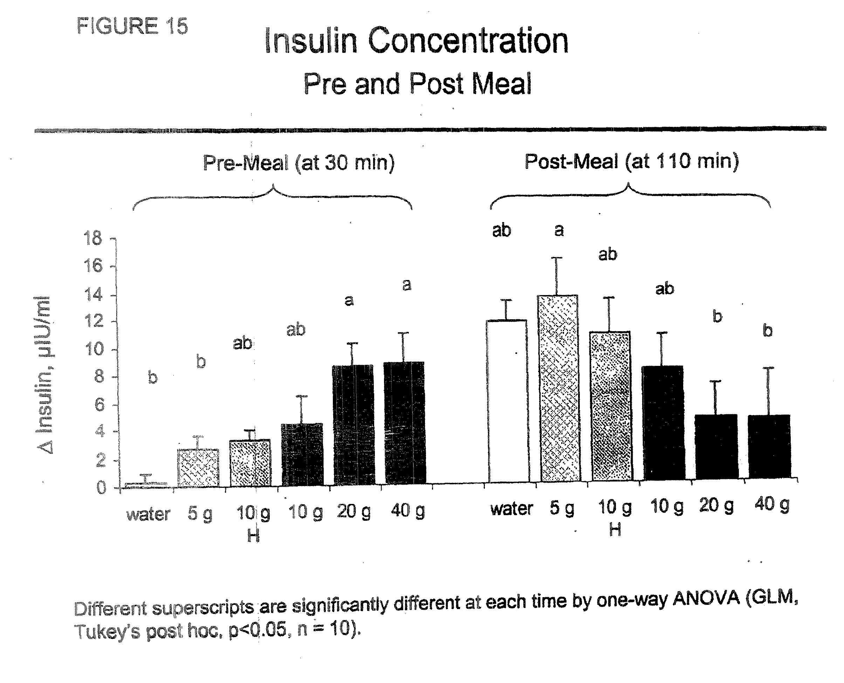

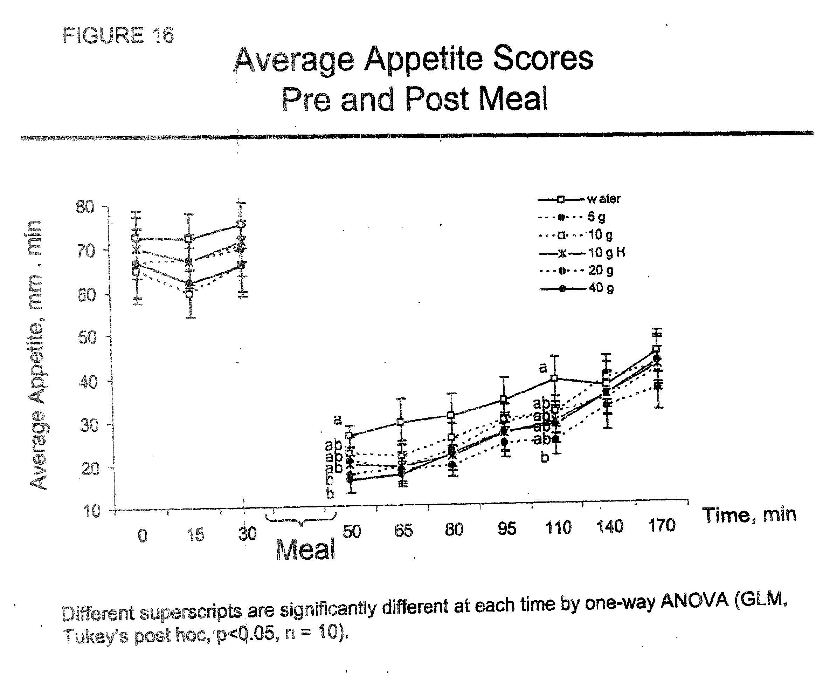

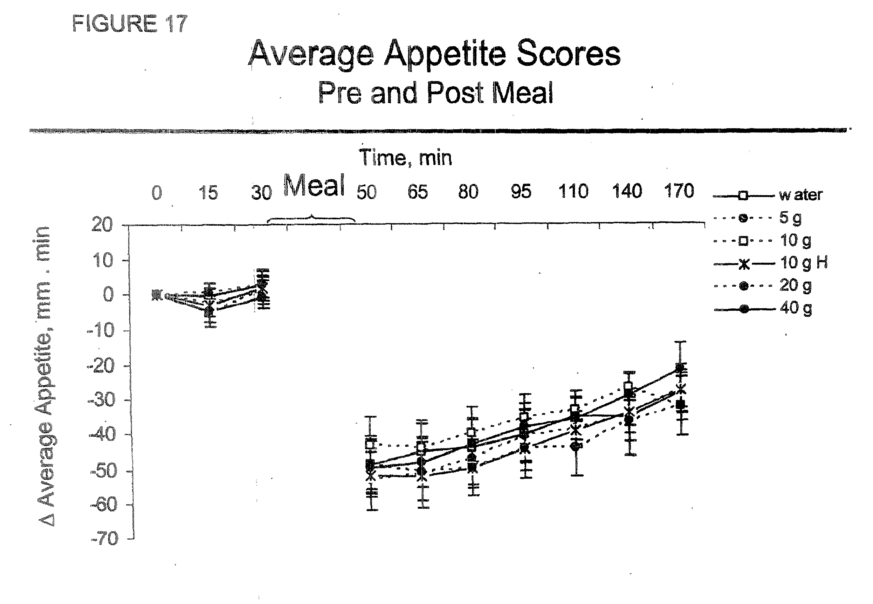

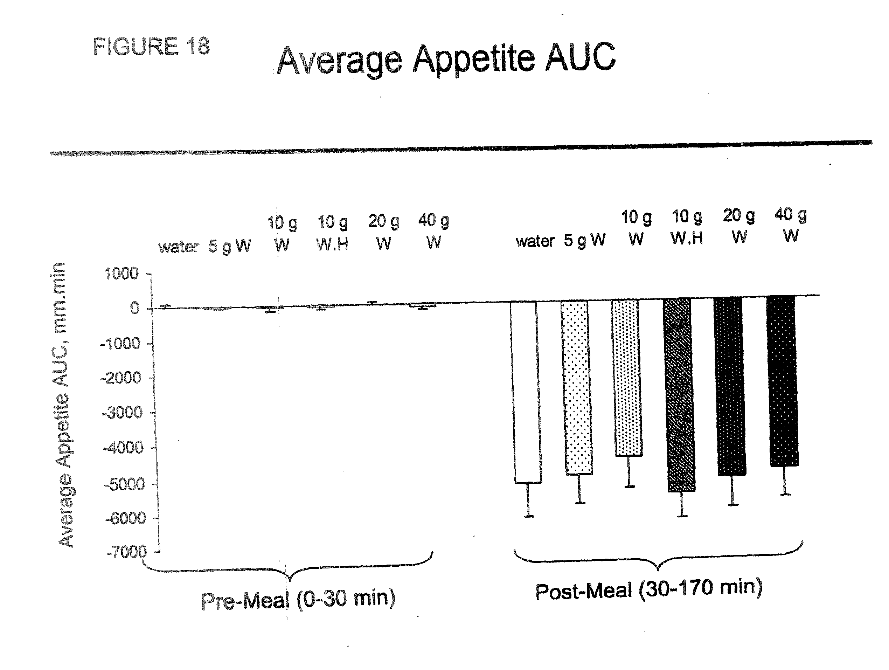

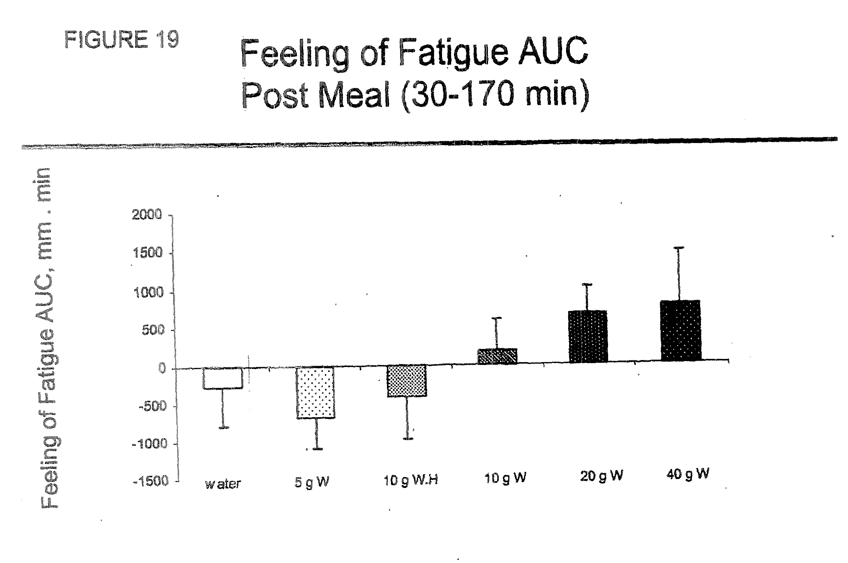

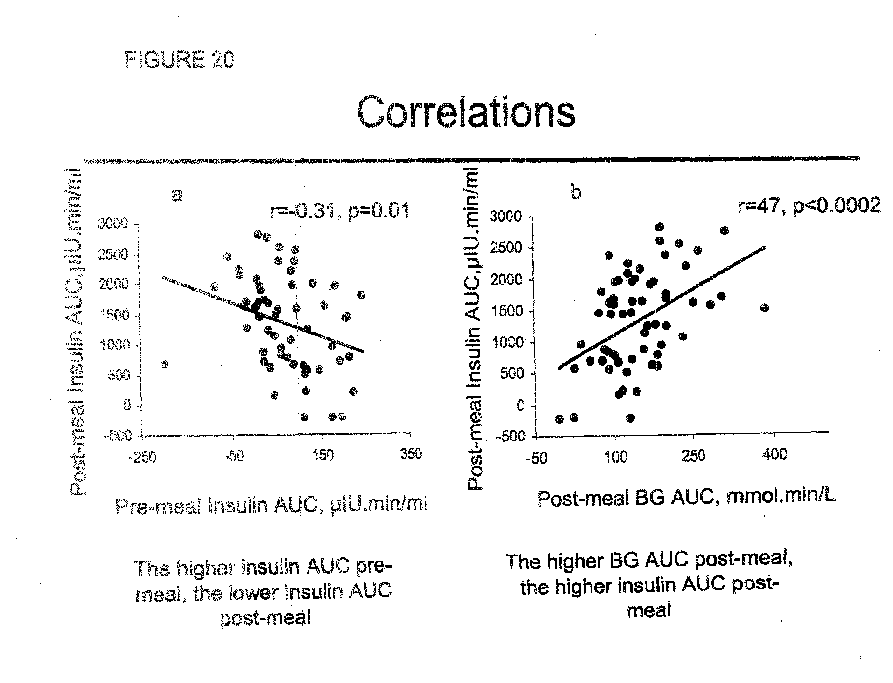

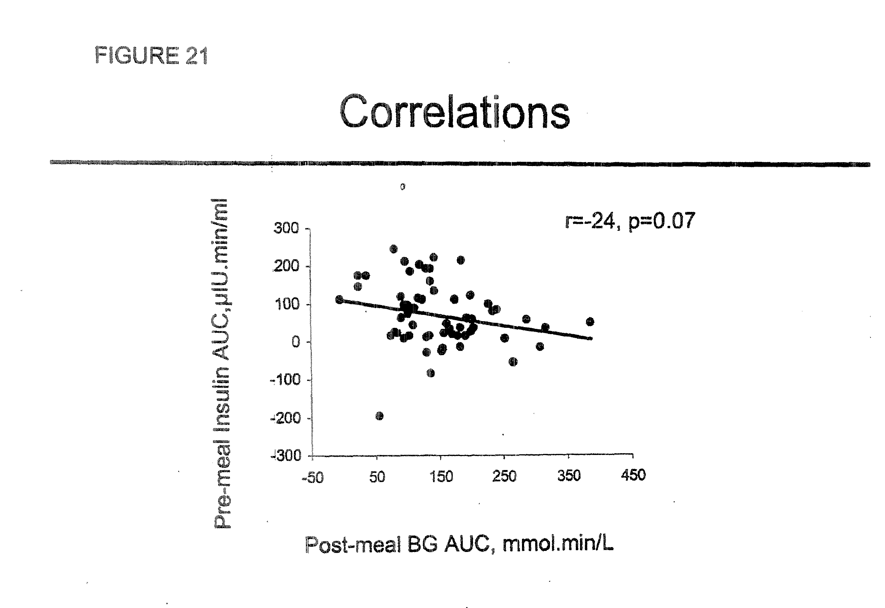

Treatment subjects randomly received the treatments at 4 hours after a standard breakfast (standard breakfast = 2% milk, orange juice and cereal for a total of 340 Kcal). Average appetite by visual analog scale (Visual Analogue Questionaire as described in Example 1) and blood glucose and insulin by finger prick blood collection were measured immediately before and at 15 and 30 minutes after receiving the treatments. Subjects then had 20 minutes to eat a test meal providing a caloric does of 12 kcal/kg body weight. At 50 minutes (right after eating the test meal), and 65, 80 and 95 minutes after receiving the treatments, blood glucose, insulin, and subjective appetite were measured. The effect of all treatments on food intake and blood glucose, and insulin are illustrated in Figures 8-21.

### EXAMPLE 3: Fixed Meal Study #2

A follow up randomized controlled crossover study was performed in a separate clinical research laboratory to determine whether a glucose-lowering effect could be observed with a lower dose of whey protein. All treatments were served in liquid form (400 ml total) that included water, drink mix sweetened with nonnutritive sweetener for color and flavor, and the amounts of sweet whey protein indicated below.

### Treatment

| No. | Treatment |
|---|---|
| 1 | 5 grams whey protein as preload |
| 2 | 10 grams whey protein as preload |
| 3 | Placebo beverage (including drink mix) as preload (control) |

Treatment subjects included 21 males and females between 21 and 51 years old with a BMI between 25.0 and 30,0.

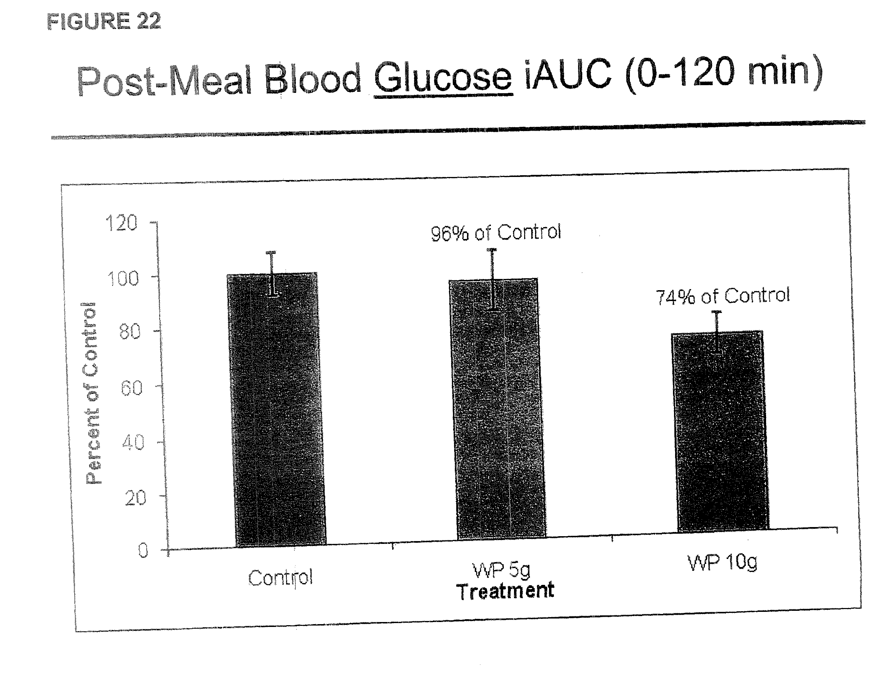

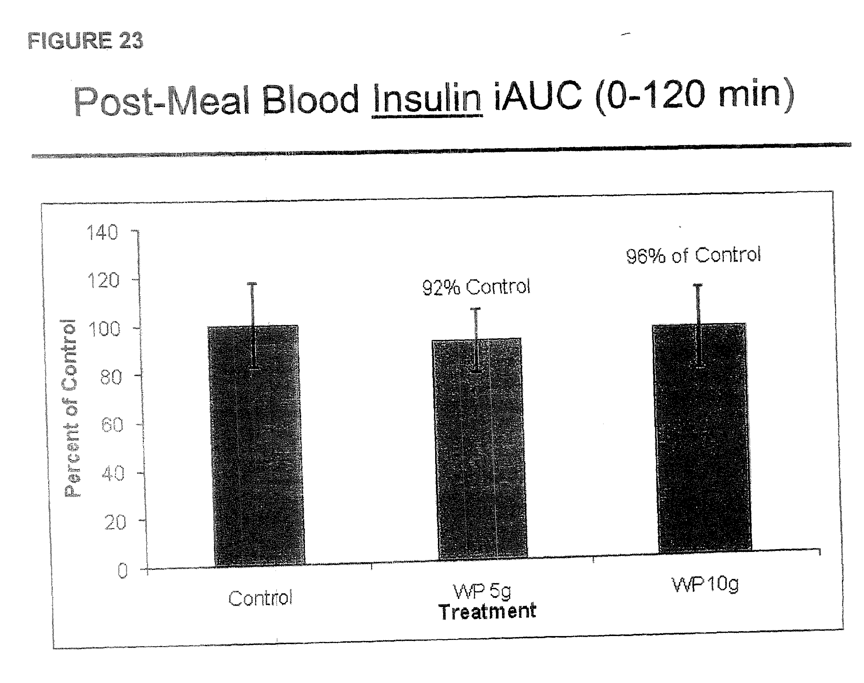

Treatment subjects randomly received the treatments after an overnight fast. Subjects were allowed 5 minutes to consume their assigned test beverage. Blood glucose and insulin by finger prick blood collection were measured immediately before and at 30 minutes after receiving the test beverage. Subjects then has 10 minutes to eat a standard meal providing 50g of available carbohydrate. At the first bite of the standard meal a timer was started and additional finger-prick blood samples were taken at 15, 30, 45, 60, 90 and 120 min after the start of the meal. The effect of all treatments on food intake and blood glucose, and insulin are illustrated in Figures 22 and 23.

## Claims

1. A method for reducing postprandial blood glucose levels comprising:
administering from 2 to 90 grams of native whey protein to a subject at least 5 to 90 minutes prior to a meal, the method effective for reducing postprandial blood glucose levels by at least about 5 % as compared to the same subject not administered native whey protein prior to a meal.

2. A method for reducing food intake comprising:
administering from 2 to 90 grams of native whey protein to a subject at least 5 to 90 minutes prior to a meal, the method effective for reducing next meal caloric intake by at least about 10% and as compared to a subject not administered native whey protein prior to a meal.

3. The method of Claims 1 or 2 wherein the native whey protein has a level of hydrolysis of 2 or less.

4. The method of any one of Claims 1 to 3 wherein the native whey protein is administered about 20 to about 40 minutes prior to a meal.

5. The method of any one of Claims 1 to 4 wherein the native whey protein is administered about 30 minutes prior to a meal.

6. A method for reducing postprandial blood glucose levels and/or food intake comprising:
administering an edible composition that includes from 10 to 90 grams of native whey protein to a subject at least 5 to 90 minutes prior to a meal, the method effective for reducing next meal caloric intake by at least about 10 % and postprandial blood glucose levels by at least about 5 % as compared to a subject not administered native whey protein prior to a meal.

7. The method of Claim 6 wherein the edible composition that includes native whey protein is selected from the group consisting of a beverage, a nutritional supplement, a food composition, a meal replacement product, and mixtures thereof.

8. The method of Claim 7 wherein the beverage is selected from the group consisting of water, fruit juice, a low calorie fruit flavored beverage, coffee, tea, smoothie, a dairy beverage, a carbonated beverage, and mixtures thereof.

9. The method of Claim 7 wherein the nutritional supplement is selected from the group consisting of vitamins, minerals, trace elements, phytochemicals, and mixtures thereof.

10. The method of Claim 7 wherein the food composition is selected from the group consisting of dairy based products, soy based products, breads and cereal based products, cakes, cookies, biscuits, spreads, oil-in-water emulsions, ice creams, desserts, soups, powdered soup concentrates, sauces, powdered sauce concentrates, health bars, confectionery, snack foods, ready-to-eat meal products, pre-packed meal products, dried meal products, and mixtures thereof.

11. The method of any one of Claims 1 to 10 wherein the native whey protein is selected from the group consisting of sweet whey, acid whey, individual whey proteins and mixture thereof.

12. The method of any one of Claims 1 to 11 wherein the native whey protein is administered in an amount of 2 to 40 grams per subject.

13. The of any one of Claims 1 to 12 wherein the native whey protein is administered about 30 minutes prior to a meal.
